# EUROPEAN PATENT SPECIFICATION

(11) **EP 1 551 286 B1**
(45) Date of publication and mention of the grant of the patent: **22.11.2017**
(21) Application number: 03808197.2
(22) Date of filing: 09.10.2003
(51) Int. Cl.: A61B 5/04, A61B 5/08, A61M 16/10

(54) **APPARATUS FOR MAINTAINING AND MONITORING SLEEP QUALITY DURING THERAPEUTIC TREATMENTS**
GERÄT ZUR ERHALTUNG UND BERWACHUNG DER SCHLAFQUALITûT BEI THERAPEUTISCHEN BEHANDLUNGEN
APPAREIL DE MAINTIEN ET DE SURVEILLANCE DE LA QUALITE DU SOMMEIL PENDANT DES TRAITEMENTS THERAPEUTIQUES

(30) Priority: 09.10.2002 US 417445 P; 10.10.2002 AU 2002951984
(43) Date of publication of application: 13.07.2005
(73) Proprietor: Compumedics Medical Innovation Pty Ltd, Abbotsford, Victoria 3067 (AU)
(72) Inventor: BURTON, David, Camberwell, Victoria 3124 (AU); ZILBERG, Eugene, Sandringham, Victoria 3191 (AU)
(74) Representative: Handsome I.P. Ltd
(86) International application number: PCT/US2003/032170
(87) International publication number: WO 2004/032719

(56) References cited:
- EP-A- 0 702 978
- WO-A-01/56631
- WO-A-97/16216
- WO-A-98/50095
- WO-A-02/065901
- WO-A-02/087433
- US-A- 5 476 483
- US-B1- 6 431 171
- US-B1- 6 488 634
- US-B1- 6 502 572

## Description

### Field of the Invention

Generally, the invention relates to the field of therapeutic treatments. More specifically, the invention relates to an apparatus for delivering therapeutic treatments to patients without adversely affecting their sleep.

### Background of the Invention

Many therapeutic treatments are administered to a patient while they are sleeping or are attempting to fall asleep. While these treatments may achieve their intended result, they also often severely affect the quality of sleep that the patient gets while undergoing these treatments. These treatments often interrupt the patient's normal progression of sleep, causing transient arousals. While these arousals do not result in the awakening of the patient, they often pull patients from deeper stages or higher quality states of sleep. Patients often do not reenter these deeper stages of sleep for a relatively long period of time.

In some instances, a therapeutic treatment may cause numerous arousals. This fragments the patient's sleep and prevents the patient from reaching the deeper stages of sleep. Studies have shown that fragmented sleep results in excessive daytime sleepiness. This, in turn, is a direct contributor to many accidents, to a general feeling of lethargy, deterioration of cognitive performance, and/or daytime sleepiness, in the patient.

One example of therapeutic treatments causing sleep fragmentation is in the treatment of sleep disorders. Continuous Positive Air Pressure (CPAP) treatments are a primary remedy for a number of sleep disorders such as sleep apnea, hypopnea, and snoring. CPAP treatments consist of delivering a constant positive airway stream of air pressure into a patient's airway during sleep in order to keep the patient's airway from collapsing upon itself. State-of-the-art CPAP machines, often called auto-titration PAP (APAP) machines, automatically adjust the pressure of the delivered air in order to accommodate a patient's respiratory pattern. to the rapid changes of pressure in the patient's airway caused by the APAP machines. Another drawback of current state-of-the-art APAP machines is that they are subject to either false positives (such as when UAR and/or natural irregular breathing events are not pre-empted or do not occur, despite false detection of such and associated treatment control change) or false negatives (such as when genuine upper airway resistance (UAR) and/or related events are pre-empted or do occur but are not detected or responded to with treatment control change). This is due in part to the reliance of these machines on the correct interpretation of an inspiratory waveform and the inaccuracies related to the interpretation of the underlying waveform by the APAP machine. This can also be due to current state of the art gas delivery (or other treatment control such as pacemaker devices) devices inability to enable suitable algorithms to detect and adapt their computation detection sufficiently to preempt or predict the probability or onset likelihood of shallow breathing, UAR, arousals, and or associated sleep fragmentation or sleep quality deterioration.

The inspiratory waveform varies periodically for reasons not always associated with upper airway resistance. The use of inspiratory waveform as the primary or only means of detection of UAR-related events can cause remedial auto titration measures to be taken when none should be. This is particularly evident where the inspiratory waveform analysis technique does not employ an underlying time-course computational method. The time-course computational method refers to comparing a previous sequence of breaths (prestored from previous treatment session or stored from current session breathing data) or the current breath and comparing the variations or changes as an inferred measure of arousal or sleep fragmentation onset. Excessively rapid or excessively insensitive pressure changes often occur when an auto-CPAP machine tries to correct a normal non-UAR related event, or misses detecting the presence of subtle shallow breathing, hypopnea or UAR, respectively. It is believed that the primary cause of sleep fragmentation is the rapid pressure changes in the patient airway produced by the current APAP machines.

In addition to the above, studies have also suggested that some APAP machines are limited in their ability to accurately detect the onset or incidence of shallow breathing, mild hypopnea, or UAR events. This limitation is also possibly attributed to limitations of the machines in interpreting the wave form. Misdiagnosis of such mild hypopnea events results in increased UAR which in turn results in arousal and subsequent sleep fragmentation.

Current state-of-the-art therapeutic devices do not optimally adapt to minimize arousals during therapy. Each patient's arousal threshold is affected by varying parameters, yet current state of the art devices do not have adaptive control algorithms that can adapt their treatment levels to accommodate a number of these varying parameters. These varying parameters include (but are not limited to) sleep history such as sleep deprivation or sleep propensity, physiological factors, psychological factors including (but not limited to) stress or anxiety, environmental factors including temperature; noise; lighting; vibration, factors such as varying threshold to arousals with changing age, drugs and alcohol effects to arousal thresholds and others.

Consequently, in light of the inherent drawbacks in current therapeutic methods for administering treatments to patients who are sleeping or are attempting to sleep, there exists a need for an apparatus and method of monitoring for patient arousal and for adapting a therapeutic treatment to minimize arousal.

WO9716216A1 discloses an apparatus for controlling gas delivery to a patient, in which a digital processing means implements a pressure seek algorithm that includes an iterative process that increases pressure if a respiratory event such as snoring or obstructive apnea/hyponea is detected. The pressure may continue to be increased until the event ceases, subject to a recommended maximum pressure not being exceeded. After the event ends, the pressure is reduced.

### Summary of the Invention

For the purposes of explanation only, the present invention is described primarily in the context of controlling delivery of gas to a patient. One skilled in the art can readily appreciate that the present invention is readily adaptable for use with other therapeutic treatments. The said therapeutic treatments can include ventilatory support or assist devices, oxygen therapy devices or pacemaker devices. As such, it is not intended that this invention be limited to the control of gas delivery.

In one aspect, the invention provides a sleep monitoring and maintaining apparatus comprising an airflow sensor for detecting breathing waveform shapes of a patient, a patient gas delivery device having an adjustable gas pressure delivery level, and a controller in communication with the airflow sensor and the gas delivery device, characterised in that the controller is adapted to: monitor the detected breathing waveform shape while the gas pressure delivering level is ramped up; and apply an adaptive algorithm for detecting variation in the monitored detected breathing waveform shape that is indicative of a probable onset of arousal and, in response to said detecting variation in the monitored detected breathing waveform shape that is indicative of a probable onset of arousal, maintain or reduce the gas pressure delivery level to avoid arousal until the monitored detected breathing waveform shape indicates the patient is in a deeper level of sleep than indicated by the detected variation in the monitored detected breathing waveform shape that is indicative of a probable onset of arousal, enabling continued ramping up of the gas pressure delivery level, wherein the variation detected by the algorithm is associated with standard empirical data threshold settings or where available previous patient data, including a specific airflow shape characteristics or various threshold indications, thus indicating the probable onset of arousal. Embodiments of the present invention may be found in the dependent claims, to which reference should now be made.

The present invention is capable of maintaining the sleep quality of a patient undergoing a therapeutic treatment by sensitizing the therapeutic device to various physiological indicators which predict the onset of arousal and using an adaptive algorithm to modify a patient's therapeutic treatment. The therapeutic control algorithm of the present invention has the capability to be adapted during real-time operation based on any combination of a) empirical clinical data, b) individual patient collected or alternative (to laboratory) collected data (from diagnostic study within sleep laboratory or other alternative site) or c) real-time monitored and analyzed data.

In one embodiment, the present invention has a capability to apply empirical clinical data to establish standard threshold configurations, which in turn determine a therapeutic device's response and performance given the current condition of the patient. In the case of a gas delivery device, parameters such as the rate of pressure change, the absolute amount of pressure change, the minimum delivered pressure values and the maximum delivered pressure values can be used. In order to minimize arousals while maintaining the integrity of the treatment, these rates and absolute pressure changes are adjusted in accordance to various patient states including (for example only) the patient's current sleep state or the patient's relative blood pressure or arrhythmia detection. The present invention can be configured to rely on a fixed set of reference data designed to predict the onset or detect the occurrence of arousal.

In one embodiment, the present invention is capable of operating with or without any previous patient data. In the case where a subject has no previous data or threshold indications, the present invention could commence operation with standardized empirical data threshold settings. During device generated pressure changes, or whenever there is a respiratory disturbance or prediction of onset of a respiratory disturbance, the present invention can adapt its control characteristics to minimize the respiratory and arousal disturbance. Control characteristics refer to the rate and absolute pressure changes delivered to a subject together with the devices sensitivity to detect subtle hypopnea, shallow breathing, or UAR. Respiratory disturbance, arousal or upper airway resistance can be detected with an airflow shape monitor, or more comprehensive combinations of physiological monitored channels. In the simplest configuration the present invention would record and note the likelihood of arousal or upper airway flow limitation by way of the shape characteristics of the airflow signal (as derived from a breathing mask circuit). This detection of waveshape characteristics could be achieved by detecting changes in the sequence (1 or more) breathing waveform shapes and then associating these changes with the onset probability or actual incidence of hypopnea, shallow breathing or UAR.

In one embodiment, the present invention includes an algorithm for detecting variation in airflow shape that could be indicative of the incidence or probable onset of upper airway resistance (UAR) or variations of UAR, respiratory event related arousals (RERA) or treatment event related arousals (TERA). These airflow shape variations (and others) can be detected in the breathing mask of a patient undergoing CPAP, oxygen concentration, ventilation or other gas delivery or ventilation support. The detection capability of airflow shape variations enable the present invention to adopt analysis techniques such as neural networks or other methods that are capable of adopting self-learning and algorithm adaptation techniques.

In one embodiment, self-learning and adaptation techniques are specifically applicable to the detection of RERA and TERA. RERA and TERA can be detected by monitoring cortical or subcortical activity or by detecting airflow wave shapes associated with generation of such RERA's. Alternatively, airflow and shape only analysis methods can be adopted.

In one embodiment, the present invention is adapted to detect UAR, RERA, and TERA in a patient using physiological parameters such as pulse transit time (PTT) pulse arterial tonometry (PAT), plethysmographic wave amplitude, electroencephalogram (EEG), electro-myogram (EMG) and electro-oculogram (EOG), to name a few.

Utilizing these techniques, a gas delivery pressure device (oxygen concentrator, ventilator, VPAP, CPAP, APAP and others) can predict the UAR, RERA and TERA events or the onset of such events and adjust the treatment to avoid such events.

In one embodiment, the process of detecting and monitoring for arousals could occur simultaneously or in virtual real-time with automated gas delivery treatment algorithms which are able to adapt to reduce or eliminate both sleep breathing disorders and sleep fragmentation. The present invention is able to recognize when the pressure adjustment of the gas delivery device is either too severe and leading to the promotion of RERAs or TERAs or avoid the failure to compensate for less obvious (without comprehensive shape analysis and possibly patient specific calibration) or more subtle SBD such as UARs, hypopnea events, and shallow breathing.

### Brief Description of the Drawings and Figures

For purposes of facilitating and understanding the subject matter sought to be protected, there is illustrated in the accompanying drawings an embodiment thereof. From an inspection of the drawings, when considered in connection with the following description, the subject matter sought to be protected, its construction and operation, and many of its advantages should be readily understood and appreciated.
Fig. 1 is a schematic diagram of one embodiment of the present invention.
Fig. 2 is a schematic diagram of the arousal monitoring functions of the present invention..
Fig. 3 is a flowchart of the airflow diagnostic process for the present invention.
Fig. 4 is an example of a waveform for inspiration cycle with snoring.
Fig. 5 is an example of a waveform for an inspiration with a UAR.
Fig. 6 is a flow diagram of one embodiment of the present invention.
Fig. 7 is a schematic diagram of one embodiment of the present invention.

### Detailed Description of the Preferred Embodiments

### A. General Overview

The present invention is an apparatus and method for maintaining the sleep quality of a patient undergoing a therapeutic treatment. The present invention monitors and interprets physiological signals and spontaneous breathing events to detect the onset of arousal. Once the onset of arousal is determined, the present invention determines adjustments that are needed in the operation of a therapeutic device to avoid or minimize arousals.

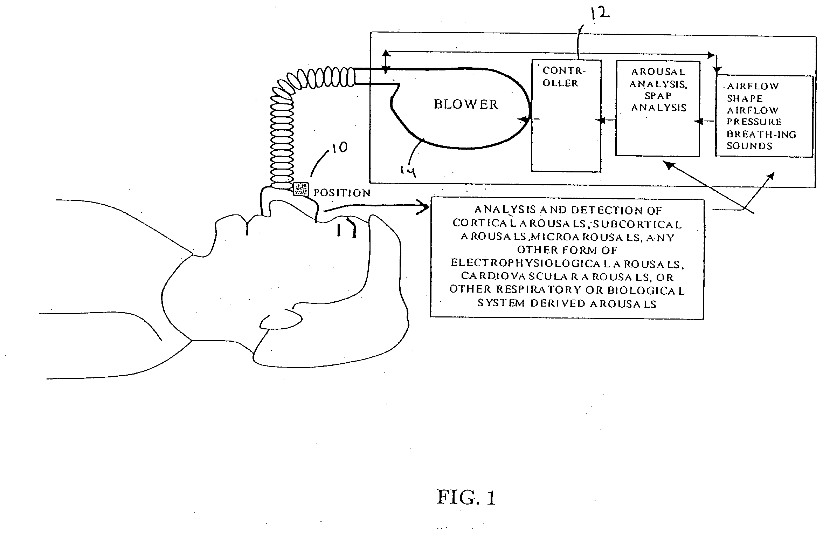

As shown in Fig. 1, the apparatus includes one or more sensors 10 which detect a patient's physiological parameters, a controller 12 which monitors and determines arousal based on the physiological variables received from the sensor, and a gas delivery apparatus 14 which is controlled by the controller 12. The sensor 10 can be a combination of one or more devices which are able to monitor a physiological parameter that is used by the present invention to determine the onset of arousal or the onset of a sleep disorder. The sensors can be integrated into one unit or may operate independent of the others. The apparatus is adapted to determine arousal using physiological parameters such as pulse transit time (PTT) pulse arterial tonometry (PAT), plethysmographic wave amplitude, electroencephalogram (EEG), electro-myogram (EMG) and electro-oculogram (EOG), to name a few. The apparatus is also adapted to monitor, analyze, and compute the sequence of airflow shape and sound. The breathing waveform profiles or sequence of waveform profiles or sounds of a patient are matched to various templates which are correlated to specific arousal events or Sleep Breathing Disorders. The presence of SBD, UAR, shallow breathing or the onset of the same, can be analyzed and computed. The apparatus may receive a plurality of inputs from sensors and match the inputs to values listed in a plurality of tables. The tables identify various breathing waveform profiles and physiological parameters or sequence of waveforms and physiological data and matches this information to a particular arousal event or sleep breathing disorder. Furthermore, a number of co-efficients and equations can be applied to the values stored in the table in order to accommodate variations which are patient specific. The apparatus may have a capability of operating in three different modes. One mode is a default mode wherein empirical data establishes thresholds and reference data used to compute optimal therapeutic control. The apparatus also includes a calibration mode wherein it tests the response of the patient to various settings in order to determine patient tolerances. The apparatus also includes an adaptation mode wherein the present invention utilizes optimal therapeutic control in order to minimize or eliminate arousal events or SBD.

### B. System Configuration

The apparatus according to the present invention includes three main components, a sensor for monitoring a physiological parameter, a therapeutic device for administering a therapeutic treatment, and a controller for controlling the delivery of the therapeutic treatment. The present invention is described as having three main components for the purposes of explanation only. One skilled in the art can readily appreciate that the three main components of the present invention can be readily integrated into one or more devices.

In one embodiment, the present invention includes a number of sensors, some of which are used to detect upper airway resistance and airflow and some of which detect physiological parameters which are used to determine arousal. The sensor can be any apparatus known in the art which is capable of detecting, measuring, or calculating a physiological parameter which is used to determine arousal. The sensor can be comprised of a single integrated machine or a plurality of independent ones. The sensors can communicate with the controller by any known protocol.

In one embodiment, pressure transducers and a pneumotachograph are used in cooperation or integration with an airtube or a patient mask to detect patient airflow and airway pressure. To detect physiological parameters, the present invention uses sensors such as, but is not limited to, EEG, EOG, EMG, ECG, pulse oximetry, blood pressure, carbon dioxide monitoring, bed transducers for monitoring patient position, video processing systems and microphones for breathing and breathing sounds.

Preferably, the sensors are all incorporated onto a single patient mask. A suitable mask is disclosed in International Publication Number WO 01/43804 entitled "Bio-mask with Integral Sensors,". The mask has sensors integrated therein which are capable of detecting EMG, EEG, EOG, ECG, surface blood pressure, temperature, pulse oximetry, patient sounds, and gas pressure in the mask. The mask can include side-stream or full-stream gas sampling capability for monitoring in real-time, concentration of oxygen, CO₂, nitric oxide and other gases or any combination of the aforesaid gases. In addition, the mask serves as the conduit for gas delivery to the patient. A mattress device may be used to detect arousal. Currently, there are two commercially available mattresses which can perform the above functions. One is known as a Static Charge-sensitive Bed (SCSB) and the other is a polyvinlidene fluoride (PVDF-piezoelectric plastic) bed. Eye activity may be used to monitor arousal. An infrared video monitoring system is employed as a sensor to determine eye activity via eyelid position. The image signal from the video monitoring is processed by graphic processing program to determine the status of the eyes. The apparatus may utilize a unique multi-standard wireless interface system. Typically, two separate wireless bands are deployed to separate physiological wireless signals from control data. Furthermore, integrated encryption and security may be deployed to avoid unauthorized access to data.

An example could be where the 2.4 GHz ISM band is applied for the interface of wireless based sensors interfaced to a controller. Less critical data, not effecting the patient therapy, such as user data viewing and reports, could be interfaced using W-LAN and even Bluetooth™ wireless devices. The multi-radio standard is a particularly important consideration where operating with existent wireless systems. A further capability is to detect interference from similar radio band system and switch the critical signal and other monitoring either to an alternate band or modify the system analysis adaptation without wireless signals. The present invention can be used with a range of wireless electrode devices to enable easy expansion and access to additional physiological signals. The sensors may be battery powered for 1 or 2 days while transmitting signals to the controller. The wireless monitoring capability enables the present invention to monitor RERA, TERA, and SBD-related (sleep breathing disorder) signals during a subject's sleep. Furthermore, the ability to monitor these electrodes during a subject's sleep might give some augmented information (in addition to the respiratory airflow, pressure and sound signals normally derived from the subject's mask) and the ability of the SPAP system to provide optimal therapeutic pressure or gas delivery control to minimize RERA or TERA, while also minimizing obstructive sleep apnea-hypopnea (OSAH) and UARs.

This comparison of sleep efficiency during routine CPAP or enhanced (additional wireless signals applied, for example) CPAP operation can provide valuable information to the healthcare worker and patient in terms of sleep efficiency options for the patient. In a similar manner the patient may choose to utilize a wireless position sensor which could be attached to the therapeutic breathing mask or other parts of the patient therapeutic equipment or clothing.

The said wireless electrode contains several key functions enabling this wireless technology to be used with relative trouble-free ease within the patient's home or the clinical environment alike. The electrodes can be packaged such that the removal of the disposable electrode outer package activates the battery. This automatic wireless electrode activation function enables automatic preservation of the battery life, particularly during storage. Use-by dating of the disposable electrode packaging ensures that both the electrode quality and battery life is used within a suitable period of time, protecting the user from battery age deterioration and electrode deterioration. The wireless interfaced electrodes can be provided with self-gelled properties to simplify electrode attachment. The disposable self-adhesive (or reusable) electrode systems can be attached by the patient using simple visual guides.

The sensors input their physiological data to the controller (incorporates preprocessing required for treatment control), which receives the data and determines arousal or onset of arousal. The controller may include an analog processing circuit which converts analog signals from the sensors into a digital signal. The analog processing circuit utilizes known preamplifying, amplifying, conditioning, and filtering configurations to enable the analog sensor signal to be converted into a digital signal. In some instances, the sensor may directly input a digital signal.

In one embodiment, the controller also includes a processor which receives the digital signal and determines the patient state and an appropriate setting for the gas delivery or other therapeutic device. The processor employs a plurality of tables stored in a database. The tables include a plurality of entries which correlate the inputted signal from a sensor with arousal. Typically, a number of different physiological parameters are inputted simultaneously and all of the parameters are factored in determining arousal. The processor can employ a weighting system for each parameter and the appropriate action is determined by the derived index value. In another embodiment, the processor can link a chain of physiological values together and compare it to a table which correlates the linked set of values to arousal.

In one embodiment, the present invention includes memory devices containing tables holding stored profiles containing normal or acceptable limits for physiological parameters such as:
sleep fragmentation, apnea-hypopnea index (AHI), RERA, sleep architecture, cortical arousals, sub-cortical arousals, PTT values, PAT values, HRV values, central sleep apnea (CSA) occurrence, apnea occurrence, mixed apnea occurrence, hypopnea occurrence, EEG spike occurrence, EEG spindle occurrence, EEG K-complex occurrence, EEG seizure occurrence, bi-coherence or bispectral index values, auditory evoked potential index, patient posture optimal pressure values, patient sleep propensity, patient sleep state.

The controller communicates with the therapeutic device to control the treatment level to the patient. The controller determines an appropriate instruction set by using treatment levels found in the table entry corresponding to the patient's physiological condition. The processor then communicates the instruction set to the gas delivery apparatus which then executes the instruction set.

### B. Arousal Monitoring

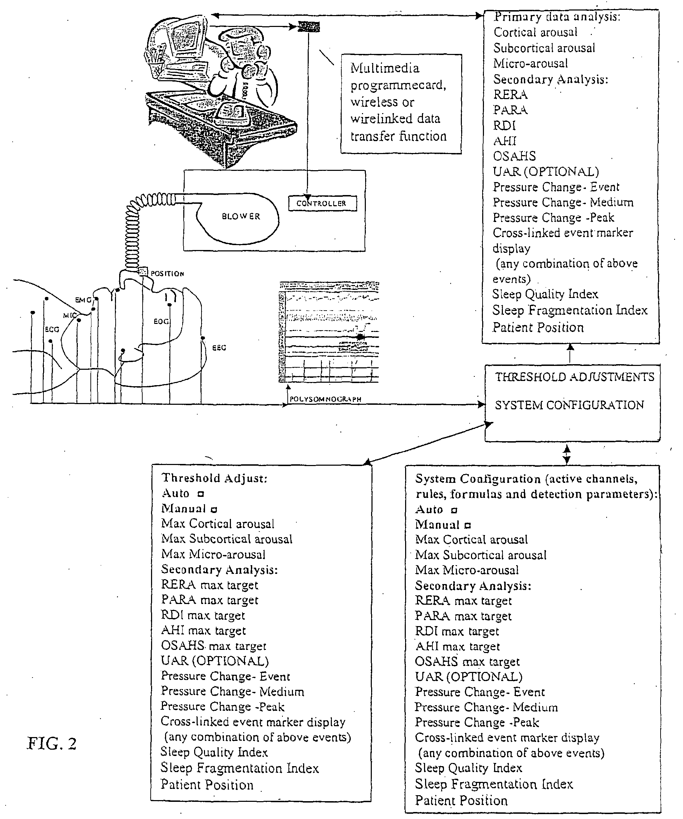

As shown in Fig. 2, the apparatus uses various physiological inputs to determine arousal in a patient and to tailor the delivery of air to the patient to minimize such arousal. Due to the complex and varying states of sleep and broad range of sleep disorders that can be diagnosed, many different physiological parameters may be monitored and analyzed to determine arousal.

The minimization of arousals includes the capability to automatically adjust the therapeutic treatment while monitoring at least one physiological parameter or signal where the monitored physiological parameter(s), signal(s) or measures can include (but are not limited to):
Blood pressure, patient movement, patient vibration, patient tremor, patient shake, Pulse oximetry, pulse-wave, EEG, EOG, EMG, patient position, patient movement, breathing sounds, airflow signal, respiratory effort signal(s), pharyngeal pressure signals, expired PC0₂ signal, diaphragmatic EMG, transthoracic impedance, electrocardiogram (ECG), reflective oximetry, pulse oximetry, oxygen saturation, nasal pressure, airflow pressure, breathing mask airflow, breathing mask pressure, breathing mask sound, breathing sound, breathing pressure, respiratory inductive plethysmography, plethysmographywave, oesophageal pressure, nasal cannular sensor signals, nasal and oral cannular sensor signals, oral cannular sensor signals, thermocouple sensor signals, thermistor sensor signals, PVD temperature sensor signals, PVD sound and vibration sensor signals, PVD breathing or airflow signals, Pneumotach calibrated flow, or other routine or research application of polysomnograph (PSG) monitoring sensors electrodes or signals. Arousals may be monitored using an EEG. Typically, the forebrain is monitored to determine cortical arousals and the brainstem is monitored to measure subcortical arousals. The onset of arousal is characterized by bursts of higher frequency EEG signals or a shift to alpha or theta activity from a slower background frequency, and, occasionally, transient increase in skeletal muscle tone. Standard EEG electrode placements and protocols may be used to measure arousals. The apparatus includes the capability to distinguish a periodic leg movement (PLM) related arousal from a respiratory related arousal. Distinguishing PLM arousals from arousal associated with respiratory events can be important, particularly where optimum treatment control may not respond to a PLM related arousals but may need to respond to a respiratory event related arousal.

The apparatus may detect and distinguish PLM and/or PLM arousals by means of comparing sub-cortical arousals inferred from blood pressure variations with cortical arousals (EEG). Cortical arousals are used to distinguish sleep-fragmentation and neurological related arousals versus sub-cortical arousals which generally include both sleep-fragmentation and neurological related arousals and PLM related arousals.

In one embodiment, the onset of arousal is determined using Pulse Transit Time (PTT). Studies have shown that sleep disorders such as apnea, hypopnea or upper airway resistance result in an accompanying arousal, and this arousal is accompanied by changes in heart rate, a transient burst of sympathetic activity, and a surge in blood pressure. Obstructive sleep apnea can be correlated with an obvious and measurable increase in intrathoracic pressure associated with obstructive effort and cardiobalistogram effect. The cardiobalistogram effect is created when the lungs apply pressure to the heart. This compresses the heart and reduces the volume of blood pumped by the heart. These cardiovascular changes are recognizable by way of a transient but significant dip in the patient's baseline PTT value.

PTT is the time taken for the pulse wave to travel between two arterial sites. The blood pressure is directly proportional to the speed that the arterial pressure wave travels. A rise in blood pressure relates to faster pulse wave and thus shorter PTT. Conversely, a drop in blood pressure results in a slowing of the pulse wave and an increase in PTT. PTT may be obtained using sensors located on the above-mentioned bio-mask. A sensor receives input from the mask and generates a plethysmography waveform. A second sensor receives input from the mask and generates an ECG signal. The waveform and the signal are inputted into the controller and a PTT reading is calculated.

The PTT is derived by utilizing a plethysmography waveform obtained by using pulse oximetry techniques in combination with an ECG signal. The ECG R or Q wave can be used as the start point for the PTT measurement and the end point of the PTT measurement can be the point representing 25% or 50% of the height of the maximum pulse wave value. EMG measurements may be used to detect levels of sleep in a patient. EMG monitoring enables the present invention to detect sleep-related changes in a patient's muscle tonicity. Sleep states will typically be accompanied by changes of tonicity in certain muscles. Arousals will typically result in increased muscle tonicity. ECG and an EMG signal from the diaphragm may be monitored in combination to detect respiratory effort associated with central apnea versus obstructive apnea. The ECG electrodes are configured on the patient in order to distinguish diaphragm related respiratory effort from thoracic respiratory effort. During central apnea, there will be a cessation of breathing without respiratory effort. This is distinctly different from obstructive apneas wherein muscle activity increases as a result of increased breathing effort to overcome the obstructed airway. A patient's eye movements may be monitored to assist in determining arousal. One technique involves the use of digital video recording and known graphic processing techniques to determine eye lid activity (i.e. whether the eye lids are closed, open, or degree of openness). Arousals may be detected by monitoring the presence of waveform signal disturbance evident on a high bandwidth analysis (DC to 200 Hz or higher bandwidth) of the airflow waveform and pressure waveform obtained within a breathing mask. Apnea events, shallow breathing, upper airway resistance and hypopnea events can also be detected and pre-empted by analysis of the change in shape of the high bandwidth monitoring of the airflow waveforms and pressure waveforms.

In addition to monitoring arousal, in one embodiment, other physiological parameters may be monitored to determine the patient's physical state. The present invention can utilize sensors in the biomask to determine heart rate, ECG, respiration rate, snoring sounds, airflow, air pressure, and O₂ saturation. Conventional methods may also be incorporated into the present invention to monitor blood pressure, and CO₂. The patient's sleeping position may also be monitored using pressure transducers or a mattress device.

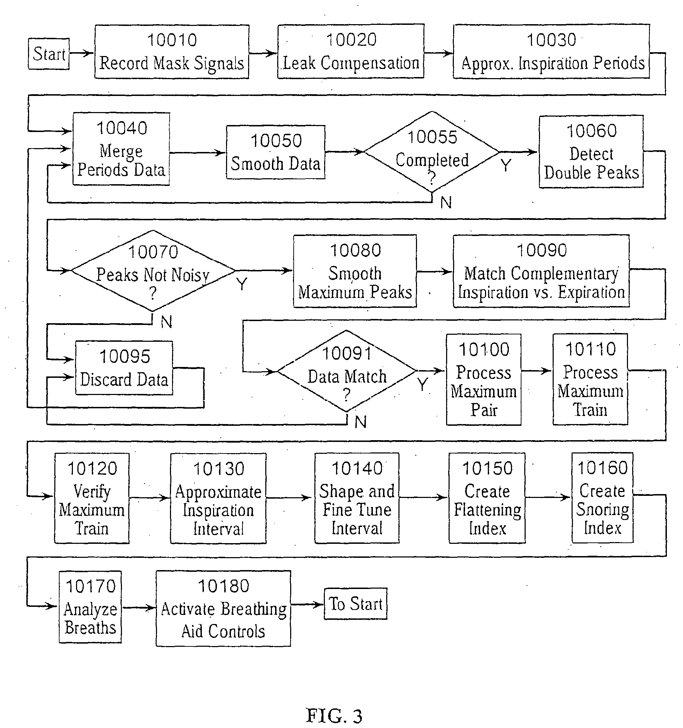

In one embodiment, wherein a patient is undergoing CPAP treatment, arousal monitoring also includes monitoring pressure and airflow associated with a patient's breathing in order to determine UAR (which may induce RERA). To prevent RERA, it is necessary to detect a number of patterns which are indicative of sleep apnea symptoms, namely inspiratory flow limitation (flattening), snoring and flow amplitude reduction (hypopneas and apneas). As detailed in Fig. 3, the present invention analyzes the airflow to and from the patient in order to determine the existence of UAR.

A "Breath detection" component performs real time detection and characterization of individual breaths. Detection of inspiration and expiration peaks includes "local" smoothing (to separate real breaths from noise) and "global" detection of respiration peaks based on relatively long context which may include up to six consecutive breaths. Breath analysis includes accurate detection of inspiration interval and characterization of flow during inspiration, namely indices of flattening, snoring and inspiratory amplitude as well as a few others.

A "Time interval based processing" component performs analysis of pressure and flow derived signals based on expiration of time intervals rather than breaths. It is necessary in cases when breaths are not discernible such as apneas or when the mask comes off the face.

The controller generates pressure adjustment signal on the basis of per breath and per time interval information provided by the two above components. The controller is implemented as a collection of rules which cover various combinations of indicators of flow limitation, snoring, breath amplitude, pressure leak and other parameters. The main strategy in breath detection is to use maximum and minimum points (flow signal level) as the indicators of inspiration and expiration intervals. Inspiration is associated with positive flow signal deflection and expiration is associated with the negative deflection. However, the flow signal could be contaminated by large amount of noise, and it is necessary to smooth flow data before detecting actual breath patterns (box 10050). For accurate detection of the inspiratory and expiratory peaks it is also necessary to use a relatively long context to prevent confusing them with local maxima and minima in the flow signal.

There are two main tasks in the local smoothing. First, all local maximum and minimum points from the flow signal are detected, and each maximum point is defined as an initial candidate for the location of an inspiration peak, and each minimum point for an initial candidate for possible location of expiration peak. The second task is to smooth some maximum and minimum points with "relatively small amplitude", which are likely to be noise signal. As a result of local smoothing, only maximum and minimum points with "relatively large amplitude" are retained, and the flow signal is considered to be sufficiently smoothed. This sequence of local smoothing be described as follows:
1. Detect all local maximum points from a set of flow data.
2. For each maximum point, form a pattern called max-peak, in which the maximum point is located in the center, and data in its left side increase monotonously, and decrease monotonously in its right side. For the current flow data set, obtain a set of max-peak patterns.
3. For the same data set, detect all local minimum points and obtained a serial of min-peak patterns using the similar method.
4. Calculate a number of parameters such as signal variation and duration for each max-peak and min-peak, and these parameters are used as the measurements to test whether some of detected max-peak and min-peak patterns are in fact noise.
5. Analysis sequences of adjacent max-peaks and min-peaks (in every sequence the number of max-peaks should exceed the number of min-peaks by one or alternatively the number of min-peaks should exceed the number of max-peaks by one) and check if a sequence could be approximated by a single max-peak or min-peak so that an approximation error is significantly less than variation and duration parameters of a resulting max-peak or min-peak
6. For the noise signal smoothing, use piecewise linear methods to approximate the flow signal.
7. The max-peaks with relative large amplitudes are retained, and for each "retained" max-peak, both 'increasing period' (left side) and 'decreasing period' (right side) are not shorter than a pre-defined threshold (0.75 s).
8. Same method is applied to min-peak smoothing processing.

The local smoothing is basically designed for excluding noise signals that have a relatively small amplitude and short duration. As a result, a large amount of maximum and minimum points can be excluded from a list of "candidates" for inspiration and expiration peaks. The local smoothing processing can form separate "increasing periods" or "decreasing periods", and the signal within an "increasing period" or a "decreasing period" corresponds to a "likelihood" of the half duration of inspiration or expiration. This "half-duration" smoothing processing is one approach for deleting small noise signal. On the other hand, the "half-duration" approach lacks capability of smoothing flow data containing some relatively large noise and artifacts.

Another difficult problem in breath detection is related to the change of respiration patterns. The flow signal is often affected by patients that change their "way" of breathing, in other words, some periods of the increasing or decreasing signal level are related to change of patient's respiratory "behavior" rather than to inspiration or expiration. Local smoothing is unable to exclude these types of max-peaks or min-peaks. However, it is possible to use some global measurements to effectively detect these "unlikely" max-peaks or min-peaks, and this is the idea behind the global detection of respiratory peaks (global smoothing) (box 10080). In the global smoothing, multiple consecutive breaths are checked to further disqualify some max-peaks or min-peaks from the list of candidates for inspiration or expiration peaks.

For a relatively long time interval (up to 3.5 minutes), conditions are tested for pairs of successive max-peaks (min-peaks as well), and a set of so-called max-pairs (or min-pair) is formed. Then a number of conditions for series of max-pairs to obtain a set of max-pairs with similar 'patterns', denoted as max-train are developed. The same processing is carried out to generate min-trains out of min-pairs. Therefore there are two main parts in the global smoothing, namely generation of max-pairs (min-pairs) (box 10100) and max-trains (min-trains) (box 10110). The following paragraphs outline max-pair and max-train processing briefly (min-pair and min-train generation employ the same respective methods).

From the starting point of the max-peak set, a pair of max-peak patterns is determined, which must meet the following conditions:
(1). The duration between two max-peaks must be longer than the minimum duration of a breath (0.75 s).
(2). The duration between two max-peaks must be shorter than the maximum duration of a breath (10 s).
(3). There is not any intermediate max-peak within a max-pair. An intermediate max-peak pattern is defined as that the signal level of the maximum point in a intermediate max-peak pattern is larger than 80% of signal level of the maximum point in the max-pair itself.
This search processing is carried out through the whole max-peak pattern set to obtain a sequence of max-pairs.
(4). For each max-pair, a number of statistical measurements are calculated, and these measurements are based on the difference between the original flow signal and the approximation lines within each max-pair.

There are two main outputs in this processing, one is a set of max-pairs which is one step closer to the final set of inspiratory peaks, and another is a number of statistical measurements which is used to represent the "shape" of the max-pair. In the subsequent max-train processing, we will rely on these statistical measurements to carry out 'similarity' test.

The main method used in the max-train processing is called "similarity" test, i.e., we measure the "similarity" within a sequence of max-pairs to form a sequence of max-pairs with "similar" pattern, denoted as max-train. Only the max-pair that passes a "similarity test" can be included into the max-train. The idea behind the max-train is that
i. A sequence of normal breaths over a successive period of time (3- 6 breath durations) should have similar shapes, and this pattern should not be changed significantly over a short period of time as well.
ii. If a max-pair is not similar to this normal breath pattern, it could be rather like a respiratory event (apnea or hypopnea), or some noise/artifact pattern in flow signal.
The brief algorithm of max-train processing is then as follows:
1. Each candidate max-pair must first meet minimum duration requirement that is defined as the distance between candidate and the reference max-pair.
2. Starting from each single candidate, we calculate a number of parameters such as duration, variation of signal level, the shape of max-peak (or min-peak). We then calculate some statistical measurements for this group of candidates such as mean, deviation, average and maximum error for all the elements to check the similarity among of these candidates.
3. If the condition of the similarity is met, the group of max-pair is formed as a max-train (box 10120). Otherwise, the processing is moved into next max-pair until all max-pair are checked.
4. The same method is applied to the min-train processing.
5. Using max-train and min-train sets, we are now able to detect the locations of global maximum points of flow signal level that are related to the inspiration periods, and a number of minimum point that is associated with the expiration periods.

The global max-peak and min-peak arrays provide estimated locations of each breath, i.e., inspiration and expiration peaks. In order to detect respiratory events, one needs to closely look at these breaths, which includes:
1. Detect the start and end points of inspiration interval (box 10130).
2. Perform flow flattening (box 10150) and snoring analysis (box 10160) as well as calculation of other breath parameters.

During smoothing processing a linear approximation method is used to smooth flow data except of maximum and minimum points in max-train and min-train data sets. However, for purpose of breath analysis 'recover' raw flow data using maximum and minimum points as references is needed prior to carrying out breath analysis processing.

There are two steps involved in detecting inspiration, namely estimation and fine-tune processing. For the inspiratory interval estimation, the assumption is that the amount of intaking flow during inspiration period should be same as that of 'expiring-out' flow during expiration period. Using the maximum and minimum points in flow signal as references we estimate the interval of inspiration, i.e., the start and end points of inspiration based on calculating the areas of flow data.

However, this method has inherently two problems that could effect the accuracy in inspiration detection. Firstly, when the flow is measured at the mask the amounts of flow during inspiration period and the followed expiration period may not be the same, especially when patients use their mouth to breathe, and we call this problem as "flow imbalance". Secondly, there may be "area insensitivity" problem. When patients start inspiration the flow signal level rapidly increases, but the measurement of flow area is an integration processing that is much slower than the change of flow signals itself. In other words, the change of flow area is not sensitive enough to accurately measure the start point of inspiration where flow signal is changed rapidly.

The flow area is first calculated to estimate the inspiration interval, which includes the start point and the end point of an inspiration. The start point of an expiration period is simply defined as the end point of the previous inspiration period, and the end point of the expiration period is the start point of the following inspiration period or can be ignored as this point does not play any role in our control algorithms. Starting from the estimated start point inspiration period, linear approximation methods to detect the "break point" during flow signal increasing period, and this break point is then defined as the start point of the inspiration interval. The end point of the same inspiration period is simply defined as a point at which the signal level is the same as that of the start point of the inspiration but it has passed the maximum point.

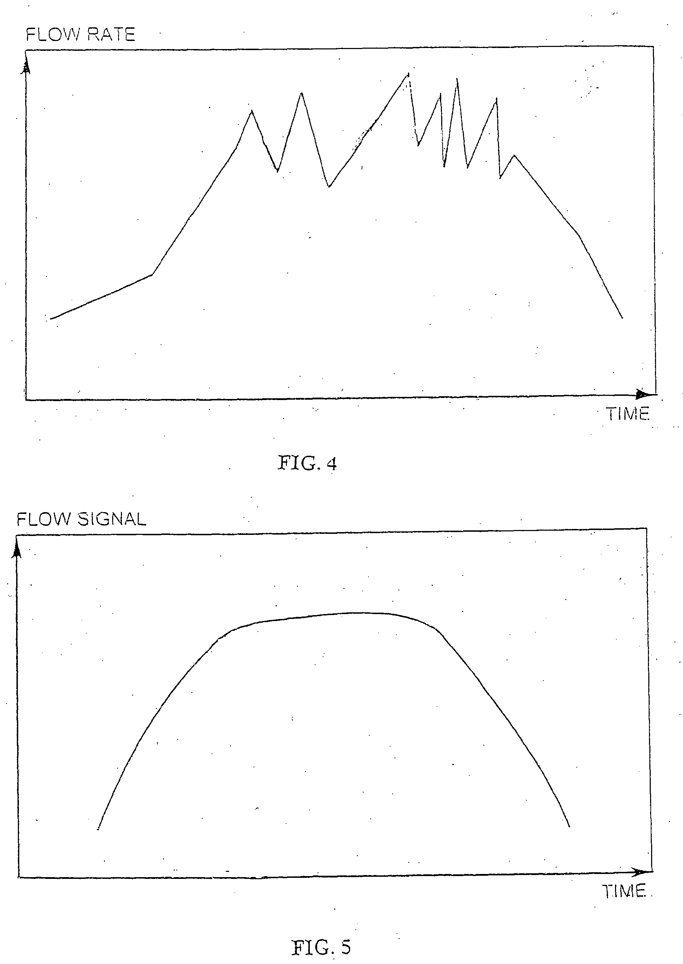

As mentioned previously, the present invention needs to detect three types of respiratory events, namely apneas and hypopneas, snoring, and inspiration flow limitation. The first type of events (apneas and hypopneas) is associated with reduction of inspiration flow and this can be resulted directly from the breath detection. Both snoring and inspiration flow limitation are more likely to occur during "abnormal" breath period. For a "normal" breath, the "shape" of signal on the top of inspiration flow appears "rounded" and relatively smooth. When snoring is present the high frequency flow signal is visible during inspiration as shown in Fig. 4. Inspiration flow limitation is defined as the event that the patient is unable to generate continuous flow increase during the first half of an inspiration period. As a result, the flow signal on the peak of inspiration flow becomes 'flat' as shown in Fig. 5. In flattening analysis, we determine a reference "flat" line which can be best fitted for the flow signal on the top of inspiration according the least square error (LSE), and the difference of the flow signal and the reference "flat" line during this period is then calculated as a flattening error. There are a number of flattening errors for different selections of the reference line. The flattening error with the smallest value is defined as a flattening index. The flattening index is then used to measure flow limitation, and the smaller the flattening index, the more severe is the inspiration flow limitation. A snoring index is also utilized to indicate the degree of the snoring. The snoring index is defined as measurement of the amount of high frequency signal on the top of inspiration flow.

### C. Operation

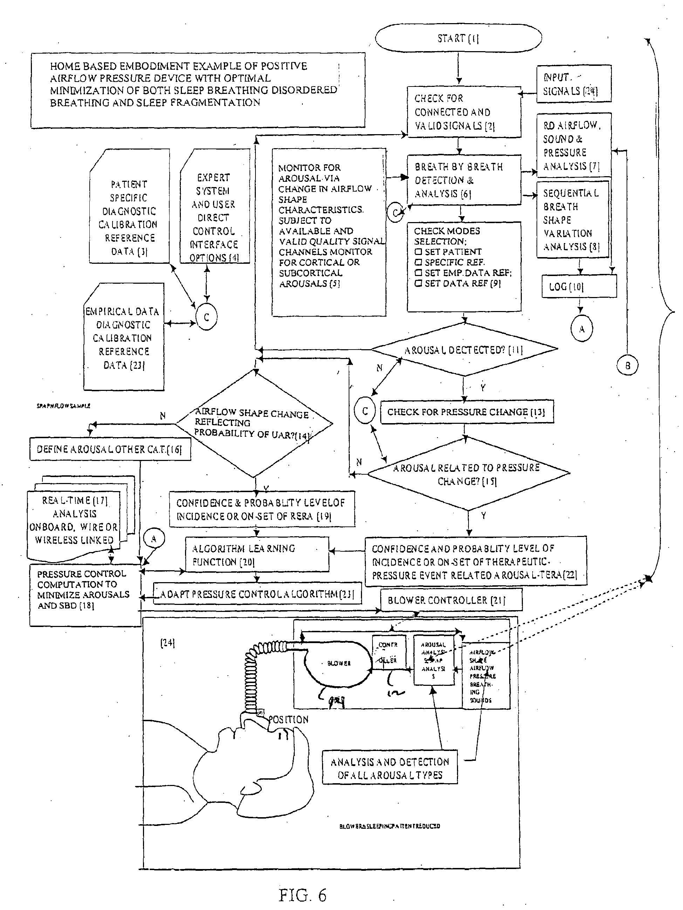

An operational flow chart of one embodiment of the present invention is shown in Fig. 6. For the purposes of explanation only, the present invention will be described in an embodiment which is adapted for use with a CPAP machine. One skilled in the art can readily appreciate that the subject invention is easily adapted for use with, or incorporated within, other known therapeutic devices.

The present invention checks to make sure that it is receiving valid signals from its sensors. (box 2) Once the signal is verified, the signals are analyzed in order to determine if the onset of arousal has been detected. (boxes 5, 6, 7, and 8). The data used in the analysis is determined by the user.

In one embodiment, the present invention has the capability to use different forced oscillation treatment (FOT) to determine patient-specific threshold values for arousals and SBD. Results from the FOT are used to create templates which are used to determine the appropriate therapeutic response to avoid the onset of or eliminate the incidence of CSA, OSA, OSAHS, RERA, and TERA. (box 3) These templates or profiles are determined from patient-specific diagnostic studies or the appropriate FOT treatment at each particular stage in a subject's sleep or breathing status.

The present invention can obtain patient-specific FOT templates and profiles by utilizing forced oscillation of pressure, or changes in airflow pressure, to determine whether the changes in the airflow shape resulting from these subtle treatment changes are able to counteract the shape or profile characteristics indicative of the incidence or on-set of arousals (TERA or RERA) or OSAH and UAR. The present invention can vary the pressure change value and rate of change to countermeasure such events.

The present system provides a means to down-load from sleep laboratory studies or other types of previous sleep, respiratory and/or cardiac related investigations. The specific data is associated with a subject's breathing and sleep arousal parameters and is used to customize a gas delivery device to be more sensitive and accurate for both minimizing incidence of UARS, OSAHS, RERAs and TERAs, while still minimizing sleep fragmentation and optimizing sleep quality. (box 23) Each patient has a unique respiratory breathing circuit and associated pathways. Subsequently breathing waveforms during all stages of sleep of a patient will vary from patient to patient. The present invention ability to accommodate the patient's personal empirical data provides a means to produce more sensitive and effective treatment algorithm. If the onset of arousal has been determined (block 11), the present apparatus then determines if the CPAP has caused a pressure change (box 13) or if the event is caused by the existence of UAR (box 14). If there was no pressure change attributed to the CPAP machine, the present apparatus will likely determine that the onset of arousal was caused by RERA or another form of arousal. If there was a CPAP related pressure change, the present apparatus would make a determination if the onset of arousal was attributed to the pressure change or some other event (box 15). An appropriate remedy will then be selected based on the based on the physiological signals and the patient respiratory flow. (box 18)

In one embodiment, the present invention is able to utilize the determination to adapt the the empirical data. (box 20) This enables the present invention to become more acutely sensitive to the physiological response of the patient.

The minimization of arousals includes the capability to automatically adjust the therapeutic treatment based on at least one index or derived data set wherein the index or derived data set can include the following:
Upper Airway Resistance (UAR), Respiratory Effort-Related Arousal (RERA), Therapeutic-control Event-Related Arousal (TERA), Respiratory Disturbance Index (RDI) Respiratory Arousal Index (RAI), Apnea-hypopnea index (AHI), Arousals (Micro-arousals), Arousals (Cortical), Arousals (subcortical), Arousals (Total), Total Arousal Time, Sleep stage, REM sleep, Sleep on-set, Body movement, Percentage of arousal disrupted sleep (breakdown of all disrupted stages), Sleep efficiency Index, Sleep Fragmentation Index (new- SFI- Total Sleep Fragmenting arousals per hour), Airflow Shape trend, Airflow Shape Template Type, Flattening Index, Forced oscillation event, Pressure change event, Pressure change rate, Pressure change event curve, Pressure change event maximal and minima, Mixed Sleep Apnea events, Central Sleep Apnea events, Upper Airway Resistance Syndrome (UARS) events, Obstructive sleep apnea and hypopnea syndrome (OSAHS) events, Respiratory Effort-Related Arousals (RERA) with screen linked qualification of associated respiratory effort arousals, Therapeutic control Related Arousals (TERA) with screen linked qualification of pressure changes and arousals, Sleep Quality Index (new- hourly sleep index factor) Quality associated arousals, Oxygen Desaturation, Pulse Transit Time (PTT), Pulse Arterial Tone (PAT), Pulse Wave Amplitude (PWA), desaturation events and SpO₂ artifacts - accurate detection of cascaded desaturations, desaturations with SpO₂ artifacts inside, SpO₂ artifact start and end positions, detection sequences of respiratory events with partial or short recovery, classification of respiratory events with noisy or poor quality effort signals, detection episodes of Cheyne-Stokes breathing, Concordinance capability to allow score comparisons between any two designated data sets, Pneumotach calibrated flow, Thermal sensor flow, Sum of respiratory effort signals, EEG Arousals, PTT, Plethysmographic wave, Transthoracic impedance, Detection and allowance screen grid highlighting for an expanded set of automatic events to include the following events, Obstructive Sleep Apnea/Hypopnea event or syndrome (OSA, OSH, OSAHS), Respiratory effort related arousal, Central Sleep Apnea (CSA), Central Sleep Hypopnea (CSH), Cheyne-Stokes breathing, Hypoventilation, Yawn, Unstable breathing related to sleep state changes or onset of deeper stages of sleep, Swallowing, Coughing, Spontaneous or irregular but normal shaped breathing signals, Derived tidal volume (from nasal pressure or calibrated flow), Derived flow limitation index (from nasal pressure or calibrated flow), Derived snoring (from nasal pressure or calibrated flow), Derived diaphragmatic EMG amplitude, Derived upper airway resistance (from mask pressure, pharyngeal pressure and calibrated flow), Derived subcortical arousals (from PTT or pleth wave amplitude), Breathing mask and/or airflow sound analysis with segmentation into various breathing disorders such as cough, wheeze, strider, apnea and hypopnea.

For every selected event (combination of a set of expanded group of events from above and current set of events), a user is allowed to select the set of measurement signals and to set the parameters of detection for the event. This enables the present invention to use more than one signal at the same time to detect an event and more than one scenario to detect an event. The following are examples of defined events:
RERA -
   1. Break in the flat inspiratory profile after a few flow limited breaths
   2. Frequency shift in EEG, amplitude increase in EMG
   3. Subsequent leg movement activity
   4. No pressure augmentation (CTRL signal)
Leg movement related arousal-
   1. Increase in leg movement activity
   2. Frequency shift in EEG, amplitude increase in EMG
   3. Break in the inspiratory profile not necessarily during inspiration
   4. No pressure augmentation (CTRL signal)
Spontaneous arousal -
   1. Frequency shift in EEG, amplitude increase in EMG
   2. No increase (or after EEG/EMG changes) in leg movement activity
   3. Break in the inspiratory profile not necessarily during inspiration
   4. No pressure augmentation (CTRL signal)
Pressure augmentation related arousal -
   1. Pressure increase according to the titration algorithm
   2. Subsequent frequency shift in EEG, amplitude increase in EMG
   3. No increase (or after EEG/EMG changes) in leg movement activity
   4. Break in the inspiratory profile not necessarily during inspiration

The present invention significantly reduces arousal by restricting the application of pressure treatment until a patient is in a stage of sleep where this pressure is not experienced or causes no adverse patient discomfort. Pressure of air delivered to a patient is ramped up or down depending upon the patient's sleep state. Pressure is ramped up slowly while physiological parameters are monitored. According to the present invention, once the physiological parameters indicate the onset of arousal (microarousal) the pressure is maintained or reduced until the patient is in deeper levels of sleep enabling continued ramping up of pressure. Pressure is also ramped downwards accordingly.

The controller 12 is implemented as a combination of rules for pressure change. Every pressure change rule specifies the magnitude and sign of a pressure change and the allowed range of pressure values within which the pressure change can be activated as well a number of additional parameters including time constants, timeouts and forced oscillation logic. Every pressure change rule is activated if a respective logical combination of its conditions is true. In one embodiment, pressure change rules are combined via logical OR - pressure changes if any single rule in the set of rules is satisfied. If more than one rule is satisfied the rule with a higher priority takes precedence.

Conditions for various pressure change rules represent a number of physiological scenarios:
Flow limitation (flattening) over a number of subsequent breaths - pressure increase
Flow limitation (flattening) and snoring over a number of subsequent breaths - pressure increase
Snoring over one or two breaths - pressure increase
Hypopneas - pressure increase (it is recommended to use additional information such as PTT, band or mattress signals to discriminate obstructive vs central hypopneas)
Detection of apnea start - start forced oscillation
Low level of upper airway conductance with forced oscillation - pressure increase (obstructive apnea detected)
No flow limitation (rounded breath shape) - gradual pressure reduction
Large leak - pressure reduction to 4 cmH2O
No airflow over 3 minutes - pressure reduction to 4 cmH2O

The present invention is capable of overcoming varying arousal dependent factors by applying adaptive algorithm techniques. The adaptive algorithm technique has the capability to apply empirical clinical data to establish standard threshold configurations, which in turn determine a device's response and performance in terms of gas delivery characteristics. The adaptive algorithm technique also has the capability to apply a set of threshold characteristics. These threshold characteristics can vary parameters such as the rate of pressure change, the absolute amount of pressure change, the minimum delivered pressure values, the maximum delivered pressure values. These rates and absolute pressure changes can vary in accordance to various states of said patient including (for example only) the patient's current sleep state or the patient's relative blood pressure or arrhythmia detection. The apparatus can be configured in a predetermined mode of operation where the algorithm adaptation function can be disabled and replaced by an algorithm that relies on a fixed set of reference data designed to predict the onset or detect the occurrence of TERA and RERA, while minimizing sleep breathing disorders. The apparatus enables medical specialists to set various thresholds, which may prevent undesirable medical conditions for each particular patient. For example, if central sleep apnea is detected in combination with an increase or undesirable change or measure in ECG, pulse-wave or arrhythmia, the operation of the gas delivery device is augmented to stabilize the patient's condition. In some instances this stabilization may include the immediate cessation of pressure delivery. During events such as central sleep apnea (cessation of breathing activated by the brain commands versus airway obstruction), for example, forced pressure delivery without airway obstruction may otherwise aggravate the subject's blood pressure or cardiac function.

The present invention is capable of operating with or without any previous patient data (a specific airflow shape characteristics or various thresholds, for example). In the case where a subject has no previous data or threshold indications the present invention could commence operation with standard empirical data threshold settings. During device generated pressure changes or whenever there is a respiratory disturbance the present invention can adapt its control characteristics to minimize the respiratory and arousal disturbance.

In one embodiment, monitoring of arousals enables the subject invention to augment a CPAP's sleep disorder detection capabilities. False negatives often occur during mild hypopnea events. There is typically such minimal airflow limitation that CPAP machines are unable to detect the breathing disorder. However, such mild events often create sufficient UAR to cause arousal in a patient. The detection of the onset of such arousals enables the present invention to initiate a corrective response from the CPAP unit, even though the CPAP is unable to detect the event.

In one embodiment, a treatment mode includes utilizing breathing pattern templates stored in the table to augment current CPAP settings. These dynamically allocated breathing pattern templates supplement the CPAP algorithm by changing the control characteristics of the CPAP unit. The templates satisfy the particular patient's pressure requirements while optimizing the patient's sleep and minimizing patient arousal.

In one embodiment, the present invention enables the commencement of treatment to be determined by a pre-defined state of sleep, arousal activity level, and/or pre-determined sleep disordered breathing activity. One of the difficulties experienced by patients with existent state of the art gas delivery treatment devices is the discomfort experienced from the positive air pressure applied to a patient, while they are attempting to fall asleep.

Existent state of the art devices have the capability to provide a delayed start function. This delay function provides a time delay before the treatment pressure slowly increases or ramps up to a prescribed value of start pressure. However, patients are not always able to predict their sleep onset time as drowsiness of a patient varies from one night to the next. The concept of a prescribed delay time can also provide a psychological anxiety, as the patient is always aware that if they do not succumb to a satisfactory sleep state in an appropriate amount of time, they risk experiencing the unpleasant sensation of excessive positive air pressure during their sleep preparation time.

The present invention enables the detection of sleep state and/or arousals as a mean to determine pressure activation. Treatments are applied only when the patient is in a preselected or deep state of sleep and subsequently is oblivious to the said commencement of treatment. The determination of sleep state can be the methods which are known in the art, including those methods disclosed in the U.S. Patent No. 6,397,845. The apparatus may have an integrated diagnostic and treatment mode where adjustments to the delivered air are changed in real time (i.e. changes are instantaneously made depending upon the values of the monitored parameters).

The present invention's control algorithm has the capability to be adapted during real-time operation based on any combination of a) empirical clinical data, b) individual patient collected or collected data (from diagnostic study within sleep laboratory or other alternative site or c) real-time monitored and analyzed data.

### D. Alternative Embodiments

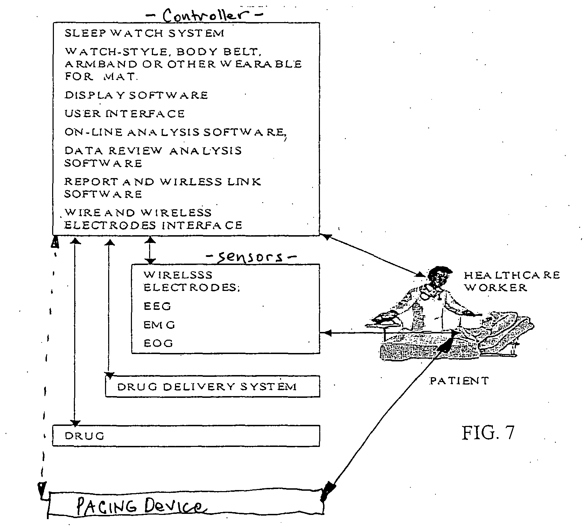

In one alternative embodiment, not part of the present invention as shown in Fig. 7, the apparatus is used to deliver medication to a patient. Previous methods for determining sedative or tranquilizer dosage requirements for a subject are often estimated on a generalized patient group or a specific sample patient group.

A subject's sleep or vigilance propensity is highly complex and dependent on many parameters. It has been shown, for example that a person's sleep propensity can be related to sleep deprivation, alcohol, anxiety, stress, environmental factors, body mass index, gender, hereditary and other factors.

The consequence of over-sedation include increased recovery time, attention deficit risks associated with excessive drowsiness, increased costs of drugs, and reduction in the quality of life due to the extended drowsy state of a subject.

The drug administration can deliver a range of drugs utilizing methods such as (but not limited to) orally, transdermal, fluid drip delivery, vapor delivery and gas delivery. Utilizing the integration of a drug delivery system with the apparatus a drug dosage can be optimized for a predetermined level or an appropriate level of drowsiness, vigilance or attention state.

The user or health-care provider could adjust drug administration dosage in consultation with the patient and the monitored patient data.

A further capability of the present invention is to contain sensors such as sensitive movement devices that together with signal analysis (such as but not limited to spectral, phase and amplitude) can detect shaking, tremors and other signs indicating appropriate drug usage. In the case of Parkinson's and other disease types the present system could be programmed to administer, for example, adequate drugs to minimize tremors and shaking, while at the same time provide the subject with a degree of vigilance during the day and sleep quality during the night that is most conducive to each individual's quality of life requirements or desires.

The present invention can be adapted in a number of configurations with different combination of physiological recording channels, sensors, analysis, storage and display capabilities. These capabilities could vary subject to the specific disease or disorder being treated, along with each subject's specific health-specialist requirements for information.

In another alternative embodiment, not part of the present invention as shown in Fig. 7, the apparatus includes a pacemaker control algorithm which minimizes RERA and TERA while optimizing a subject's heart pacing. The apparatus enables the detection of RERA and TERA from the electrocardiogram or pulse-wave signals, for example. Alternatively, more comprehensive signals can be deployed. The apparatus enables the conventional optimization of ECG pacing while at the same time minimizing arousals and sleep fragmentation. Pacemaker control can also be utilized to assist in the elimination of some sleep disordered breathing. The apparatus can provide important feedback as to the causation of sleep fragmentation such as inappropriate pacemaker control causing promotion of sleep fragmenting arousals.

The present apparatus is also able to monitor heart rate, blood-pressure variations and sleep fragmentation arousals throughout sleep and determine whether these said variations relate to normal sleep physiology, or whether these changes suggest modification of pacemaker control in order to optimize heart function, while at the same time minimizing sleep fragmentation.

In another embodiment, the present invention has the capability to provide optimal sleep during oxygen concentration treatment by utilizing cortical, subcortical, airflow shape or waveform characteristics as a marker for optimizing the treatment. The present invention can control the titration algorithm to minimize RERA and TERA while optimizing a subject's breathing therapy. The present invention enables the detection of RERA and TERA by monitoring any combination of breathing mask or hose sounds, airflow or pressure signals. Alternatively more comprehensive signals can be deployed. SOC enables the conventional optimization of blood-gas status of a subject, while minimizing arousals. An inappropriate mixture of oxygen and air, or rate of delivery of gas to a subject could promote arousals, for example.

Inappropriate gas delivery could in turn cause mechanical or chemical receptors within the patient's breathing anatomy to activate sleep fragmentation arousals (TERA). The monitoring of the airflow wave shape can be used to predict the onset or incidence of TERA or RERA and allow the gas treatment to be controlled in such a manner to minimize such arousals (while still optimizing breathing therapy).

In one embodiment, the present invention is utilized as purely a diagnostic tool for determining sleep disordered breathing and sleep quality. The present invention is adapted to record, meter, index or display, in real time or on a replay or review basis, a number of sleep or arousal related physiological data or statistics. Statistics and indexes such as RAI, AHI, RERA, RDI, arousals, sleep fragmentation, or sleep architecture index are derived from the monitored parameters and this information is stored for analysis.

In one embodiment, monitored physiological parameters monitored which were utilized to determine the statistics and indexes may also be stored in order to assist in the analysis. The present invention can also include graphical and statistical tools which are known in the art to enable a user to manipulate and display raw data or derived values in a meaningful format. In one embodiment, the present invention has the capability of displaying raw data and then using visual clues to mark the occurrence of an event such as arousal in the raw data. The present invention can also link an event or events to specific index values or derived values which reflect the occurrence of the event.

The matter set forth in the foregoing description and accompanying drawings is offered by way of illustration only and not as a limitation. The actual scope of the protection sought is intended to be defined in the following claims.

## Claims

1. A sleep monitoring and maintaining apparatus comprising an airflow sensor (10) for detecting breathing waveform shapes of a patient, a patient gas delivery device (14) having an adjustable gas pressure delivery level, and a controller (12) in communication with the airflow sensor (10) and the gas delivery device (14), the controller (12) being adapted to:
monitor the detected breathing waveform shape while the gas pressure delivering level is ramped up; and to:
apply an adaptive algorithm for detecting variation in the monitored detected breathing waveform shape that is indicative of a probable onset of arousal wherein the variation detected by the algorithm is associated with standard empirical data threshold settings or with available previous patient data, including a specific airflow shape characteristics or various threshold indications, thus indicating the probable onset of arousal,
**characterised in that** the controller is further adapted to in response to said detecting variation in the monitored detected breathing waveform shape that is indicative of a probable onset of arousal, maintain or reduce the gas pressure delivery level to avoid the probable onset of arousal until the monitored detected breathing waveform shape indicates the patient is in a deeper level of sleep than indicated by the detected variation in the monitored detected breathing waveform shape that is indicative of a probable onset of arousal, enabling continued ramping up of the gas pressure delivery level.

2. The apparatus of claim 1, wherein the gas delivery device is any one of a CPAP machine, a ventilator, and an oxygen concentrator.

3. The apparatus of any preceding claim, further comprising a memory device containing a table correlating the values of a plurality of physiological parameters to onset of arousal.

4. The apparatus of any preceding claim, further comprising a memory device containing a table correlating onset of arousal with an appropriate gas pressure delivery level.

5. The apparatus of either one of claims 3 and 4, wherein the memory device is adapted to store values for physiological parameters that indicate arousal for a particular patient.

6. The apparatus of any preceding claim, wherein the controller (12) is also adapted to detect variation in the breathing waveform shapes that is indicative of probable onset of obstructive sleep apnoea or hypopnea.

7. The apparatus of any preceding claim, wherein the controller (12) is also adapted to perform a test varying gas pressure delivery level to determine the sensitivity of a patient to treatment related arousal.

8. The apparatus of any preceding claim, wherein the controller (12) includes a processor adapted to test a patient's sensitivity to arousal.

9. The apparatus of any preceding claim, further comprising graphical and statistical tools for displaying raw data and derived indexes from the physiological parameters according to user selected formats.

10. The apparatus of any preceding claim, wherein the airflow sensor communicates with the controller utilizing a wireless technology.

11. The apparatus of any preceding claim, wherein the controller (12) is adapted to predict the onset of arousal resulting from RERA or from TERA.

12. The apparatus of any preceding claim, wherein the controller (12) is also adapted to monitor sleep quality.

13. The apparatus of any preceding claim, wherein the controller (12) is also adapted to create an index value from a plurality of different physiological signals and to adjust the gas pressure delivery level based on the index value.

14. The apparatus of any preceding claim, further comprising a further sensor selected from one or more of an EEG sensor, an EMG sensor, and a body position sensor.

15. The apparatus of any preceding claim, further comprising an ECG sensor and an Sp02 sensor, and wherein the controller includes a processor adapted to calculate PTT.

## Patentansprüche

1. Vorrichtung zur Überwachung und Aufrechterhaltung des Schlafs, umfassend einen Luftstromsensor (10) zur Feststellung der Wellenform des Atems eines Patienten, eine Vorrichtung (14) zur Abgabe von Gas an den Patienten, welche ein einstellbares Gasdruckabgabeniveau aufweist und eine Steuervorrichtung (12), die in Verbindung steht mit dem Luftstromsensor (10) und der Gasabgabevorrichtung (14), wobei die Steuervorrichtung (12) eingerichtet ist, um:
die festgestellte Wellenform des Atems zu überwachen, während das Gasdruckabgabeniveau hochfährt und einen adaptiven Algorithmus anzuwenden, um Änderungen in der überwachten festgestellten Wellenform des Atems zu erkennen, welche einen möglichen Beginn eines Aufwachens anzeigen, wobei eine durch den Algorithmus festgestellte Änderung festgelegten Schwellenwerten aus empirischen Standarddaten zugeordnet wird oder verfügbaren früheren Patientendaten, einschließlich Charakteristika einer spezifischen Luftstromform oder verschiedenen Schwellenangaben, welche somit einen möglichen Beginn des Aufwachens anzeigen,
**dadurch gekennzeichnet, dass** die Steuervorrichtung weiterhin eingerichtet ist, um, in Antwort auf dieses Feststellen einer Änderung in der überwachten festgestellten Wellenform des Atems, die einen möglichen Beginn des Aufwachens anzeigt, das Gasdruckabgabeniveau aufrecht zu erhalten oder zu reduzieren, um den möglichen Beginn des Aufwachens zu vermeiden, bis die überwachte festgestellte Wellenform des Atems anzeigt, dass der Patient sich in einem tieferen Niveau des Schlafs befindet als durch die festgestellte Änderung in der überwachten festgestellten Wellenform des Atems angezeigt wird, die einen möglichen Beginn des Aufwachens anzeigt, wodurch ein fortgesetztes Hochfahren des Gasdruckabgabeniveaus ermöglicht wird.

2. Vorrichtung nach Anspruch 1, bei der die Gasabgabevorrichtung ausgewählt ist aus CPAP-Maschinen (continuous positive airway pressure), einem Ventilator und einem Sauerstoffkonzentrator.

3. Vorrichtung nach einem der vorhergehenden Ansprüche, weiterhin umfassend eine Speichervorrichtung enthaltend eine Tabelle, welche die Werte für eine Mehrzahl von physiologischen Parametern dem Beginn des Aufwachens zuordnet.

4. Vorrichtung nach einem der vorhergehenden Ansprüche, weiterhin umfassend eine Speichervorrichtung enthaltend eine Tabelle, die den Beginn des Aufwachens mit einem geeigneten Gasdruckabgabeniveau korreliert.

5. Vorrichtung nach einem der Ansprüche 3 oder 4, bei der die Speichervorrichtung eingerichtet ist, um Werte für physiologische Parameter zu speichern, welche das Aufwachen eines bestimmten Patienten anzeigen.

6. Vorrichtung nach einem der vorhergehenden Ansprüche, bei der die Steuervorrichtung (12) auch eingerichtet ist, um eine Änderung in der Wellenform des Atems festzustellen, welche einen möglichen Beginn einer obstruktiven Schlafapnoe oder einer Hypopnoe anzeigt.

7. Vorrichtung nach einem der vorhergehenden Ansprüche, bei der die Steuervorrichtung (12) auch eingerichtet ist, um eine testweise Änderung des Gasdruckabgabeniveaus durchzuführen, um die Sensitivität eines Patienten für eine Behandlung in Verbindung mit einem Aufwachen festzustellen.

8. Vorrichtung nach einem der vorhergehenden Ansprüche, bei der die Steuervorrichtung (12) einen Prozessor umfasst, welcher eingerichtet ist, um die Sensitivität eines Patienten für eine Behandlung in Verbindung mit einem Aufwachen zu prüfen.

9. Vorrichtung nach einem der vorhergehenden Ansprüche, weiterhin umfassend graphische und statistische Hilfsmittel für eine Anzeige von Rohdaten und von den physiologischen Parametern abgeleiteten Indizes gemäß vom Anwender ausgewählten Formaten.

10. Vorrichtung nach einem der vorhergehenden Ansprüche, bei der der Luftstromsensor mit einer Steuervorrichtung in Verbindung steht, unter Verwendung einer drahtlosen Technologie.

11. Vorrichtung nach einem der vorhergehenden Ansprüche, bei der die Steuervorrichtung (12) eingerichtet ist, den Beginn eines Erwachens in Folge von RERA (respiratory effort related arousal) oder TERA (therapeutic-control event-related arousal) vorherzusagen.

12. Vorrichtung nach einem der vorhergehenden Ansprüche, bei der die Steuervorrichtung (12) auch eingerichtet ist, die Schlafqualität zu überwachen.

13. Vorrichtung nach einem der vorhergehenden Ansprüche, bei der die Steuervorrichtung (12) auch eingerichtet ist, einen Indexwert aus einer Mehrzahl unterschiedlicher physiologischer Signale zu kreieren und das Gasdruckabgabeniveau basierend auf dem Indexwert einzustellen.

14. Vorrichtung nach einem der vorhergehenden Ansprüche, weiterhin umfassend einen weiteren Sensor ausgewählt aus einem oder mehreren aus der Gruppe EEG-Sensoren, EMG-Sensoren und Körperstellungs-Sensoren.

15. Vorrichtung nach einem der vorhergehenden Ansprüche, weiterhin umfassend einen ECG-Sensor und einen Sp02-Sensor, wobei die Steuervorrichtung einen Prozessor umfasst, der eingerichtet ist, die PTT (pulse transit time) zu berechnen.

## Revendications

1. Appareil de surveillance et de maintien de sommeil comprenant un capteur de flux d'air (10) pour détecter des motifs de forme d'onde de respiration d'un patient, un dispositif (14) de distribution de gaz au patient, ayant un niveau de distribution de pression de gaz ajustable, et une unité de commande (12) en communication avec le capteur de flux d'air (10) et le dispositif de distribution de gaz (14), l'unité de commande (12) étant adaptée pour :
surveiller le motif de forme d'onde de respiration détecté alors que le niveau de distribution de pression de gaz est augmenté ; et
appliquer un algorithme adaptatif pour détecter une variation dans le motif de forme d'onde de respiration détecté surveillé, qui indique le début probable de l'éveil, la variation détectée par l'algorithme étant associée à des réglages de seuil de données empiriques standard ou à des données de patient antérieures disponibles, comprenant une caractéristique de motif de flux d'air spécifique ou diverses indications de seuil, indiquant ainsi le début probable de l'éveil,
**caractérisé par le fait que** l'unité de commande est en outre adaptée pour, en réponse à ladite détection d'une variation dans le motif de forme d'onde de respiration détecté surveillé, qui indique le début probable de réveil, maintenir ou réduire le niveau de distribution de pression de gaz pour éviter le début probable de l'éveil jusqu'à ce que le motif de forme d'onde de respiration détecté surveillé indique que le patient est dans un niveau de sommeil plus profond que celui indiqué par la variation détectée dans le motif de forme d'onde de respiration détecté surveillé qui indique le début probable de l'éveil, autorisant l'augmentation continue du niveau de distribution de pression de gaz.

2. Appareil selon la revendication 1, dans lequel le dispositif de distribution de gaz est l'un quelconque parmi une machine de ventilation spontanée en pression positive continue, un ventilateur et un concentrateur d'oxygène.

3. Appareil selon l'une quelconque des revendications précédentes, comprenant en outre un dispositif de mémoire contenant une table mettant les valeurs d'une pluralité de paramètres physiologiques en corrélation avec le début de l'éveil.

4. Appareil selon l'une quelconque des revendications précédentes, comprenant en outre un dispositif de mémoire contenant une table mettant le début de l'éveil en corrélation avec un niveau de distribution de pression de gaz approprié.

5. Appareil selon l'une ou l'autre des revendications 3 et 4, dans lequel le dispositif de mémoire est adapté pour stocker des valeurs de paramètres physiologiques qui indiquent l'éveil pour un patient particulier.

6. Appareil selon l'une quelconque des revendications précédentes, dans lequel l'unité de commande (12) est également adaptée pour détecter une variation dans les motifs de forme d'onde de respiration qui indique le début probable d'hypopnée ou d'apnée obstructive du sommeil.

7. Appareil selon l'une quelconque des revendications précédentes, dans lequel l'unité de commande (12) est également adaptée pour réaliser un test faisant varier le niveau de distribution de pression de gaz pour déterminer la sensibilité d'un patient à l'éveil lié au traitement.

8. Appareil selon l'une quelconque des revendications précédentes, dans lequel l'unité de commande (12) comprend un processeur adapté pour tester la sensibilité à l'éveil d'un patient.

9. Appareil selon l'une quelconque des revendications précédentes, comprenant en outre des outils graphiques et statistiques pour afficher des données brutes et index dérivés à partir des paramètres physiologiques selon des formats sélectionnés par l'utilisateur.

10. Appareil selon l'une quelconque des revendications précédentes, dans lequel le capteur de flux d'air communique avec l'unité de commande en utilisant une technologie sans fil.

11. Appareil selon l'une quelconque des revendications précédentes, dans lequel l'unité de commande (12) est adaptée pour prédire le début d'un éveil résultant d'un éveil lié à un évènement respiratoire (RERA) ou d'un éveil lié à un évènement de traitement (TERA).

12. Appareil selon l'une quelconque des revendications précédentes, dans lequel l'unité de commande (12) est également adaptée pour surveiller la qualité du sommeil.

13. - Appareil selon l'une quelconque des revendications précédentes, dans lequel l'unité de commande (12) est également adaptée pour créer une valeur d'index à partir d'une pluralité de signaux physiologiques différents et pour ajuster le niveau de distribution de pression de gaz sur la base de la valeur d'index.

14. Appareil selon l'une quelconque des revendications précédentes, comprenant en outre un autre capteur choisi à partir d'un ou plusieurs parmi un capteur EEG, un capteur EMG et un capteur de position de corps.

15. Appareil selon l'une quelconque des revendications précédentes, comprenant en outre un capteur ECG et un capteur de Sp02, et dans lequel l'unité de commande comprend un processeur adapté pour calculer le temps de transit du pouls.
